(19) Europäisches Patentamt
European Patent Office
Office européen des brevets

(11) Numéro de publication: **0 186 574**
**B1**

(12) **FASCICULE DE BREVET EUROPEEN**

(45) Date de publication du fascicule du brevet: **08.08.90**
(51) Int. Cl.$^5$: **A 61 K 31/70**
(21) Numéro de dépôt: **85402465.0**
(22) Date de dépôt: **11.12.85**

(54) **Nouveau médicament contenant, comme principe actif, une adénosine substituée en N6.**

(30) Priorité: **13.12.84 FR 8419047**

(43) Date de publication de la demande:
**02.07.86 Bulletin 86/27**

(45) Mention de la délivrance du brevet:
**08.08.90 Bulletin 90/32**

(84) Etats contractants désignés:
**AT BE CH DE FR GB IT LI LU NL SE**

(56) Documents cités:
**GB-A-2 077 725**

**CHEMICAL ABSTRACTS, vol. 91, no. 3, 16 juillet 1979, page 330. no. 16735h, Columbus, Ohio, US; D.S. LETHAM et al.: "Regulators of cell division in plat tissues. Part XXVI. Glucosylation of cytokinin analogs", & PHYTOCHEMISTRY 1978, 17(12), 2053-7**

(73) Titulaire: **CENTRE D'ETUDES EXPERIMENTALES ET CLINIQUES DE PHYSIO-BIOLOGIE, DE PHARMACOLOGIE ET D'EUTONOLOGIE (CEPBEPE)**
**78, rue de la Convention**
**F-75015 Paris (FR)**
(73) Titulaire: **CENTRE NATIONAL DE LA RECHERCHE SCIENTIFIQUE (CNRS)**
**15, Quai Anatole France**
**F-75007 Paris (FR)**

(72) Inventeur: **Laborit, Henri**
**93, rue de la Santé**
**F-75013 Paris (FR)**
Inventeur: **Wermuth, Camille, Georges**
**3, rue de la Côte d'Azur**
**F-67100 Strasbourg (FR)**

(74) Mandataire: **Bloch, Robert et al**
**2, square de l'Avenue du Bois**
**F-75116 Paris (FR)**

**EP 0 186 574 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

## Description

La présente invention concerne un médicament ayant comme principe actif une adénosine substituée en $N^6$, de formule I:

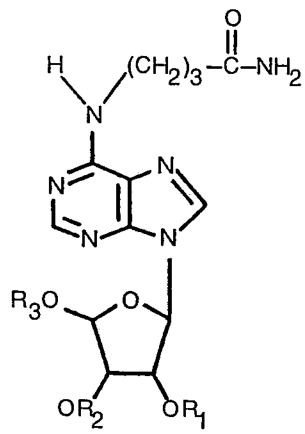

(I)

dans laquelle $R_1$, $R_2$ et $R_3$ sont identiques ou différents et représentent H ou le reste $CH_3$—$CH_2$—CO.

On connaît déjà par US—4 340 730 des adénosines substituées en $N^6$ pour le traitement général de l'hypertension, mais le médicament de l'invention s'en distingue par l'étendue de son domaine d'activité, la puissance et la durée de ses actions.

On trouvera ci-après une étude détaillée du médicament de base, désigné par la suite Agr 529 dont le principe actif est le composé de formule (I), dans laquelle $R_1$, $R_2$ et $R_3$ représentent chacun l'hydrogène.

On indiquera tout d'abord le mode opératoire de la synthèse du chlorhydrate de Agr 529.

On chauffe sous reflux pendant 10 heures un mélange de 4 g (13,2 mmoles) de chloro-6 purine riboside,, 3,6 g (26,4 mmoles) de chlorhydrate de l'amide α-amino butyrique et de 4 g (39,6 mmoles) de triéthylamine, dans 50 ml de mélange éthanol-eau (8:2).

On laisse refroidir la solution, ce qui entraîne la formation d'un précipité que l'on filtre. Ce précipité est recristallisé une première fois dans l'eau, puis dans l'éthanol absolu. On obtient 3,5 g d'un précipité blanc floconneux soit 9,9 mmoles (75%).

C.C.M.: Rf = 0,66
Plaque de cellulose F 0,1 mm
Solvant de migration $NH_3$ 10%
$H_2O$ 20%
Isopropanol 70%

p.f.: 215°C.

On dissout ces 9,9 mmmoles de $N_6$-(carboxamido-3 propyl) adénosine dans 400 ml d'isopropanol chaud auzquels on ajoute goutte à goutte 10 mmoles de HCl conc. 35% soit 0,36 g. On laisse refroidir et le chlorhydrate attendu précipite. On filtre, ce qui fournit 3 g (78%) de poudre blanche.

p.f.: 170°C.
M.A.: calculé: C: 43,35; H: 5,45; N: 21,67
trouvé: C: 43,20; H: 5.60; N:21,30
RMN: confirme la structure du produit.

La toxicité de l'Agr 529 a été étudiée sur le rat, en utilisant 7 rats mâles de 250—300 grammes, l'Agr 529 étant administré en intrapéritonéale. Le résultat des essais est donné dans le tableau ci-dessous, donnant le pourcentage de mortalité en fonction du temps.

TOXICITE DE L'AGR 529 CHEZ LE RAT

| Doses d'Agr 529 | % Mortalité à 1 heure | % Mortalité à 2 heures | % Mortalité à 6 heures | % Mortalité à 24 heures | % Mortalité à 7 jours |
|---|---|---|---|---|---|
| 500 mg/kg | 0 % | 0 % | 0 % | 100 % | - |
| 350 mg/kg | 0 % | 0 % | 0 % | 100 % | - |
| 300 mg/kg | 0 % | 0 % | 0 % | 0 % | 100 % (48 h) |
| 250 mg/kg | 0 % | 0 % | 0 % | 0 % | 0 % |

On constate que pour des doses inférieures on égales à 250 mg/kg la mortalité est nulle à 7 jours et plus.

Les doses pharmacologiques se situent entre 0,25 et 50 mg/kg.

Action anti-inflammatoire

Cette action a été testée sur l'oedème provoqué par le kaolin chez des rats mâles, Spraque-Dawley, d'un poids moyen de 350 grammes. Les animaux sont répartis au hasard dans quatre groupes, un groupe-témoin qui reçoit par voie intrapéritonéale (ip) du sérum physiologique, et trois groupes traités appelés Essais 1, Essais 2, Essais 3 qui reçoivent (ip) l'Agr 529, respectivement 5 mg/kg, 10 mg/kg et 50 mg/kg. Puis trente minutes après le traitement, tous les animaux reçoivent par voie sous plantaire 0,1 ml d'une suspension à 10% de kaolin. Le volume de la patte est mesuré à l'aide d'un pléthysmomètre (Hugo Basile 7150), une heure avant l'oedème et une, deux, quatre et cinq heures après.

Les résultats exprimés dans le tableau 1 (voir page 5) montrent qu'à la dose de 50 mg/kg, l'Agr 529 assure 100% d'inhibition à la quatrième heure et présente un action anti-inflammatoire déjà considérable pour 5 et 10 mg/kg.

Action analgésique

Cette action a été testée sur des souris mâles de trente grammes, l'Agr 529 étant administré en intrapéritonéale, et le test utilisé étant celui de la plaque chauffante.

L'appareil utilisé est un analgésimètre à plaque chauffante électrique SOCREL (Apelex). La température de la plaque est stabilisée à 56°C. On note le temps de réponse correspondant au temps nécessaire à l'animal pour se lécher les pattes antérieures ou postérieures. Pour chaque animal, le temps de réponse moyen correspond à la moyenne de trois mesures consécutives. Le test est réalisé au temps O, puis

TABLEAU 1

Evolution du volume de la patte exprimé en pourcentage du volume initial

| | Traitement | Oedème t = 0 | 1 heure | 2 heures | 4 heures | 5 heures |
|---|---|---|---|---|---|---|
| Témoins | Soluté salé isotonique 9 % | Kaolin | 15,3±2,25 | 17,35±2,4 | 20,9±2,8 (6) | 21,1±5,7 |
| Essais 1 | Agr 529 5 mg/kg | Kaolin | 8,1±2,3 | 11,0±3,3 | 9,6±3,3 (5) | 12,8±1,6 (5) |
| Essais 2 | Agr 529 10mg/kg | Kaolin | 10,1±1,3 | 9,9 ±1,3 | 8,4±1,1 (5) | 7,1± 1,55 |
| Essais 3 | Agr 529 50 mg/kg | Kaolin | 1,3±2,4 | -0,15±3,0 | 1,3±2,3 | - |

10 minutes après l'injection de la drogue, et 30 minutes après.

Les résultats sont donnés dans le tableaux 2 et 3 ciaprès (voir pages 7 et 8) qui montrent l'imporante action analgésique de la molécule.

L'action sur l'activité locomotrice

Elle a été testée sur la souris à l'aide d'une dispositif électronique à comptage automatique qui enregistre séparémment les déplacements et les mouvements de redressement de duex lots de cinq souris: mâles, toutes les cinq minutes et pendant une heure.

L'évolution de l'activité motrice est comparée à celle d'un lot témoin dont les animaux reçoivent du soluté salé isotonique dans les mêmes consitions que le lot soumis à l'action de l'Agr 529 en intrapéritonéale.

On constate qu'à partir d'une dose de 1 mg/kg, l'Agr 529 conduit à une diminution de 75% d'activité, après une heure.

La molécule de l'invention présente une action fortement hypotensive ou *anti-hypertensive*, comme le prouve notamment le fait qu'en perfusion i.v. sur le rat de 25 à 50 mg/kg, l'Agr 529 provoque un chute de tension de 40 mm de Hg. En intrapéritonéale, à la dose de 10 mg/kg, il potentialise la chute de tension due au phénobarbital i.p. en dose de 12 ou 30 mg/kg.

L'intérêt de l'Agr 529 en *anesthésie* est démontré par le fiat que chez le rat, pour une dose de pentobarbital (PB) induisant le sommeil anesthésique (30 mg/kg, i.p.), l'administration trente minutes avant, de 10 mg/kg d'Agr 529, augmente la durée de la narcose de 119%.

TABLEAU 2

| Test analgésique | Nature et doses du produit étudié | Temps moyen initial de réponse au test $t_o$ en seconde | Temps moyen de recherche au test 10 mn après injection du produit considéré $t_{10}$ en seconde | Temps moyen de réponse au test 30 mn après injection du produit considéré $t_{30}$ en seconde. |
|---|---|---|---|---|
| Plaque chauffante 56°C | Soluté salé isotonique 9 ‰ iP | $4,2 \pm 0,3$ | $4,9 \pm 0,4$ | $4,9 \pm 0,4$ |
| Plaque chauffante 56°C | AGR 529 10 mg/kg iP | $3,4 \pm 0,5$ | $18,4 \pm 2,4$ | $15,4 \pm 4,9$ |
| Plaque chauffante 56°C | AGR 529 5 mg/kg iP | $3,3 \pm 0,5$ | $8,3 \pm 1,5$ | $9,2 \pm 1,9$ |

TABLEAU 3

Etude de l'effet analgésique consécutif à l'administration

D'Agr 529 à la dose de 1mg/kg en intrapéritonéale.

| Test analgésique | Nature et dose du produit étudié | Temps moyen initial de réponse au test en S t = 0 | Temps moyen de réponse au test dix minutes après injection du produit en S t = 10 |
|---|---|---|---|
| Plaque chauffante 56°C | n=8 Soluté salé isotonique 9 ‰ i.p. | 6,1 ± 0,6 (2,5012) | +13%NS 6,9 ± 0,6 (2,4993) |
| Plaque chauffante 56°C | n=8 Agr 529 1mg/kg i.p. | 6,3 ± 0,7 (4,4479) | + 81%S 11,4 ± 1,9 (28,22) |

(Between columns 3 and 4: +13%NS for the Soluté salé row and + 81%S for the Agr 529 row. Between the two values in column 4: + 65 %.)

La comparaison des lots est faite par le test de Student avec : NS: non significatif ; et • P <0,05.

Pour une dose i.p. de PB de 12 mg/kg, qui ne fait pas perdre à l'animal son réflexe de redressement, l'injection (i.p.), trente minutes avant, de 10 mg/kg d'Agr 529 provoque une narcose comparable à celle des doses anesthésiques de P.B. (30 mg/kg).

L'étude pharmacologique permet donc de proposer, comme indications thérapeutiques à l'emploi de la molécule de l'invention, un grand nombre de désordres patholigiques.

Ont été soulignés particulièrement, plus haut, les effets anti-inflammatoires, analgésiques, tranquillisants, antihypertenseurs et anesthésiques.

Comme indiqué plus haut, le composé de l'invention se distingue des adénosines substituées en $N^6$ connues, notamment par la puissance de son action.

C'est ainsi que si l'on compare l'action sur l'activité locomotrice d'Agr 529 et du composé de formule (X)

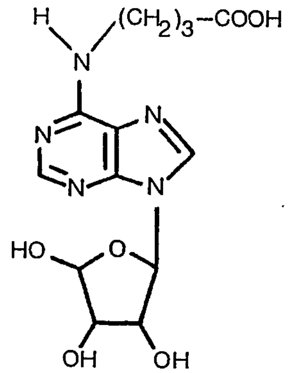

(X)

on constate que our obtenir une réduction, après une heure, de l'activité locomotrice de 75%, il faut utiliser une injection i.p. de

14 mg/kg du composé X

et 1 mg/kg d'Agr 529, qui dans ce test, parait donc 14 fois plus puissant.

De même — on a comparé (par le test de la plaque chauffante) l'action analgésique de l'Agr 529 du composé X et d'une autre adénosine substituée $N^6$, connue de formule Y

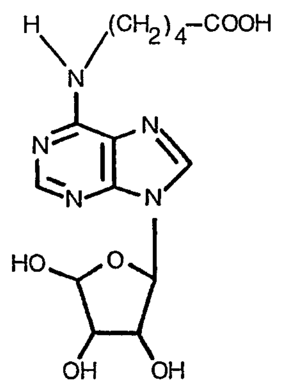

(Y)

Les résultats de l'étude comparative sont donnés dans le tableau suivant, où sont indiqués les pourcentages d'augmention du temps de réponse, 10 minutes et 30 minutes après injection de la drogue, par rapport au temps de réponse au temps 0.

| | 10 minutes | 30 minutes |
|---|---|---|
| X (70 mg/kg) | + 306,25% | + 342,85% |
| Y (73 mg/kg) | + 136% | + 230% |
| Agr 529 (10 mg/kg) | + 440% | + 353% |

On voit que l'Agr 529 a une action analgésique nettement plus forte que celle des composés X et Y et en particulier que celle du composé Y.

Outre la forme intrapéritonéale d'administration, on proposera en clinique l'utilisation des voies intra-veineuse, intra-musculaire et orale.

Les doses intra-veineuses chez le lapin s'échelonnent entre 1 et 75 mg/kg avec survie définitive. Pour l'étude sur l'homme, des doses de 0,5 à 10 mg/kg peuvent être envisagées pour l'introduction par voie intra-veineuse.

Pour la voie intra-musculaire chez l'homme, on peut penser qu'une dose intermédiaire sera utile.

Par voie orale (intubation) chez l'animal, les doses dix fois supérieures aux doses utilisées par voie i.p. sont nécessaires. Chez l'homme des doses de 50 mg/kg seront utiles.

Pour l'administration par voie orale, il est bon d'utiliser les composés de formule I dans laquelle $R_1$ et/ou $R_2$ et/ou $R_3$ représentent un reste acyle $R_4$—Co, $R_4$ représentant un reste alkyle, aryle, éthéroaryle ou aralkyle.

On obtient ainsi, à partir du composé Agt 529, des esters lipophiles des fonctions alcool primaire, qui sont des "prodrogues" conférant à l'Agr 529 une bonne activité par voie orale.

A titre d'exemple, on citera le produit de formule I dans laquelle $R_1$, $R_2$ et $R_3$ représentent le reste

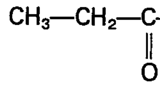

c'st-à-dire la

*(Carboxamido-3 propylamino)-6 (triproprionyl 2',3',5',ß,D ribosyl)-9 purine.*

de formule brute: $C_{23}H_{32}N_6O_8$

Ce composé est obtenu par action sur l'Agr 529 de la pyridine, en présence d'anhydride propionique.

Le mode opératoire d'obtention de ce composé est le suivant:

Dans un ballon de 100 ml introduire 25 ml de pyridine et 25 ml d'anhydride propionique. Ajouter 1 g de $N_6$-(carboxamido-3 propyl) adénosine et chauffer en agitant magnétiquement jusqu'à 80°C. Laisser à cette température jusqu'à dissolution totale de la $N_6$-(carboxamido-3 propyl) adénosine. Evaporer le mélange à sec à l'évaporateur rotatif. L'huile obtenue est purifiée par chromatographie d'elution sur silice.

Colonne Kieselgel 60

Solvant d'élution: dichlorométhane — méthanol: 90—10.

On obtient une huile jaune pâle pure en chromatographie en couche mince.

**Revendication**

Médicament ayant comme principe actife une adénosine substituée en $N^6$, de formule I:

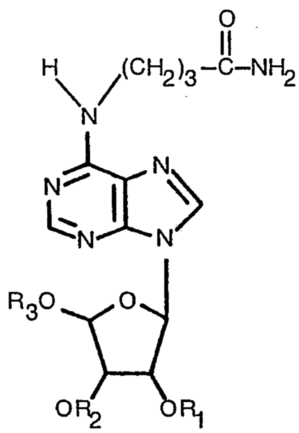

(I)

dans laquelle $R_1$, $R_2$ et $R_3$ sont identiques ou différents et représentent H ou le reste $CH_3$—$CH_2$—CO.

**Patentanspruch**

Arzneimittel enthaltend als Wirkstoff $N^6$-substituerte Adenosin er Formel I

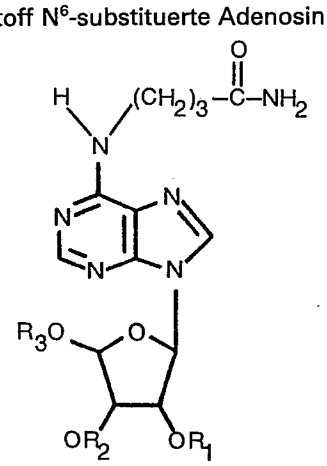

(I)

in der $R_1$, $R_2$, und $R_3$ gleich oder versehieden sind und Wasserstoff oder der Reste $CH_3$—$CH_2$—CH bedeuten.

**Claim**

A medicament comprising as active ingredient $N_6$-substituted adenosine of formula I

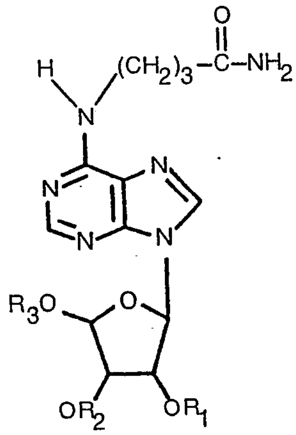

(I)

in which $R_1$, $R_2$ und $R_3$ are identical or different and represent H or the rest $CH_3$—$CH_2$—CO.